# EUROPÄISCHE PATENTSCHRIFT

(11) **EP 0 562 382 B1**
(45) Veröffentlichungstag und Bekanntmachung des Hinweises auf die Patenterteilung: **21.01.1998**
(21) Anmeldenummer: 93103978.8
(22) Anmeldetag: 11.03.1993
(51) Int. Cl.: C07D 261/18

(54) **Verfahren zur Herstellung von Isoxazol-3,4-dicarbonsäurederivaten**
Process for the preparation of isoxazole-3,4-dicarboxylic acid derivatives
Procédé de préparation de dérivés d'acides isoxazole-3,4-dicarboxyliques

(30) Priorität: 26.03.1992 DE 4209848
(43) Veröffentlichungstag der Anmeldung: 29.09.1993
(73) Patentinhaber: BASF Aktiengesellschaft, 67063 Ludwigshafen (DE)
(72) Erfinder: Maywald, Volker, Dr., W-6700 Ludwigshafen (DE); Kuekenhoehner, Thomas, Dr., W-6737 Boehl-Iggelheim (DE); Muenster, Peter, Dr., W-6823 Neulussheim (DE); Stahl, Stefan, Dr., W-6520 Worms 1 (DE)

(56) Entgegenhaltungen:
- EP-A- 0 418 667
- US-A- 3 502 660
- CHEMICAL ABSTRACTS, vol. 33, no. 21, 10. November 1939, Columbus, Ohio, US; abstract no. 8612, L. PANIZZI 'New synthesis of isoxazolepolycarboxylic acids' & GAZZ. CHIM. ITAL. Bd. 69, 1939, Seiten 332 - 339

## Beschreibung

Die vorliegende Erfindung betrifft ein Verfahren zur Herstellung von Isoxazol-3,4-dicarbonsäurederivaten der Formel I 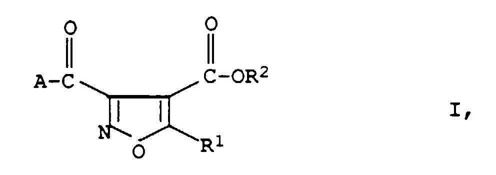 in der die Substituenten die folgende Bedeutung haben:
- R¹: Wasserstoff, ein Alkyl-, Cycloalkyl- oder Phenylrest oder ein 5- bis 6-gliedriger heterocyclischer Rest, wobei die organischen Reste unter den Reaktionsbedingungen inerte Substituenten tragen können;
- R²: Wasserstoff, ein Alkyl-, Cycloalkyl-, Benzyl- oder C₃-C₆-Alkenylrest;
- A: eine Aminogruppe NR³R⁴, in der R³ für Wasserstoff, einen aliphatischen oder cycloaliphatischen Rest steht und R⁴ einen aliphatischen, einen cycloaliphatischen oder einen ggf. substituierten Phenylrest bedeutet oder R³ zusammen mit R⁴ eine 4- bis 7-gliedrige Alkylenkette bildet, die durch Sauerstoff, Schwefel oder N-Methyl unterbrochen sein kann oder
eine Gruppe OR⁵, in der R⁵ für einen Alkyl-, Cycloalkyl-, Benzyl- oder Alkenylrest steht;
durch Deprotonierung einer CH-aciden Verbindung der Formel II 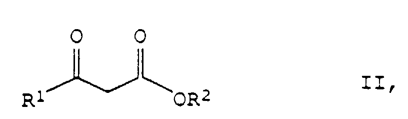 mittels einer Base und Umsetzung mit Hydroxamsäurechloriden der Formel III

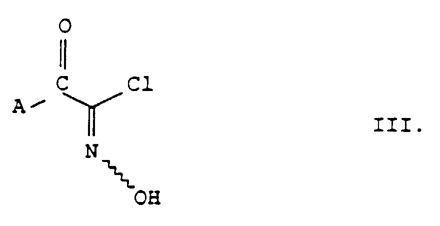

Die direkte Synthese von Isoxazol-3-carbonsäureamid-4-carbonsäurederivaten Ia 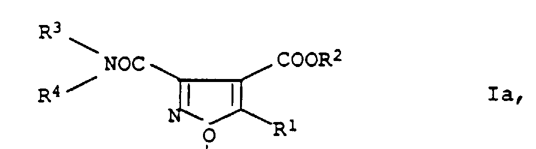 durch baseninduzierte Umsetzung von β-Ketoestern oder -säuren II mit Hydroxamsäurechloriden der Formel IIIa 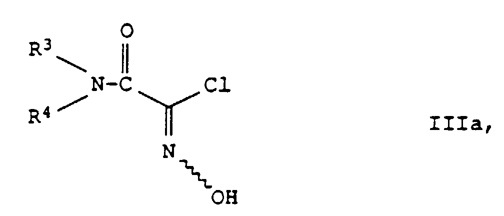 ist nicht in der Literatur beschrieben.

Literaturbekannt dagegen ist die baseninduzierte Reaktion von β-Ketoestern mit Hydroxamsäurechloriden der Formel IIIb 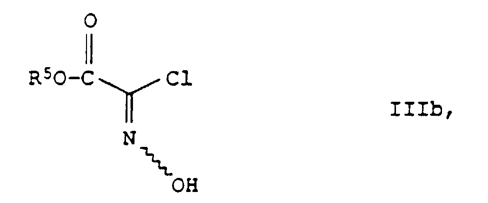 in der R⁵ Methyl oder Ethyl bedeutet (L. Panizzi, Gazz. Chim Ital., 69, 322ff (1939); V. Spiro, E. Aiello und A. Mazza, Ann. Chimica 57, Nr. 7, 836-845 (1967); V. Maywald et al., DE-A-39 31 627 sowie die Umsetzung von Alkyl-γ,γ-diethoxyacetoacetaten mit Hydroxamsäurechloriden der Formel IIIb, in der R⁵ Alkyl sowie Benzyl bedeutet (K. Butler; L.H. Conover, R.B. Woodward, U.S.-Patent 3,699,117). Bei diesen Verfahren werden jedoch entweder Natriumalkoholate oder Natriumhydrid als Base zur Überführung des β-Ketoesters in das Enolat verwendet. Nach diesen bekannten Verfahren sind die Produkte Ib 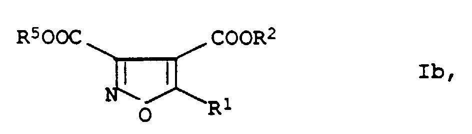 jedoch meist nur in geringen Ausbeuten zugänglich.

Der vorliegenden Erfindung lag nun die Aufgabe zugrunde, ein möglichst einfaches und wirtschaftliches Verfahren zur Synthese der Verbindungen Ia und Ib zu finden, die als Zwischenprodukte für organische Synthesen, insbesondere zur Herstellung von Pflanzenschutzmitteln (vgl. DE-A 39 31 627) Verwendung finden.

Demgemäß wurde ein Verfahren zur Herstellung von Isoxazol-3,4-dicarbonsäurederivaten der Formel I 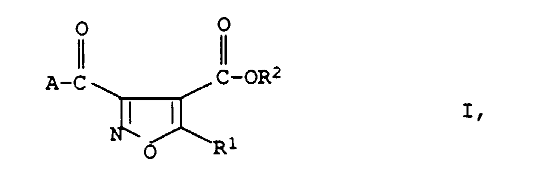 in der die Substituenten die eingangs definierte Bedeutung haben, durch Deprotonierung einer CH-aciden Verbindung der Formel II 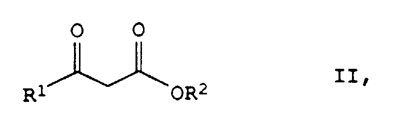 mittels einer Base und Umsetzung des Anions mit Hydroxamsäurechloriden der Formel III 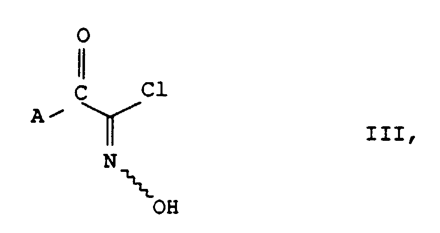 gefunden, das dadurch gekennzeichnet ist, daß man zur Deprotonierung ein Magnesiumalkoholat der Formel IV

Mg(OR⁶)₂ IV,

in der R⁶ einen aliphatischen oder cycloaliphatischen Rest bedeutet, verwendet.

Als aliphatische Reste in der Bedeutung R⁶ kommen ggf. substituierte Alkyl- oder Alkenylgruppen in Betracht. Beispiele sind C₁-C₆-Alkyl, insbesondere C₁-C₄-Alkyl wie Methyl, Ethyl, Propyl, Isopropyl, Butyl, Isobutyl, sek-Butyl, tert.-Butyl, das einmal durch C₁-C₄-Alkoxy wie Methoxy, Ethoxy, Propoxy, Isopropoxy, Butoxy, Isobutoxy, sek-Butoxy, tert.-Butoxy, insbesondere Methoxy und Ethoxy, C₁-C₄-Alkylthio wie Methylthio, Ethylthio, Propylthio, Isopropylthio, Butylthio, Isobutylthio, sek-Butylthio, tert.-Butylthio, insbesondere Methylthio und Ethylthio, Halogen wie unter R¹ genannt, insbesondere Fluor und Chlor, C₃-C₆-Cycloalkyl wie unter R¹ genannt, insbesondere Cyclopropyl, Cyano oder Phenyl substituiert sein kann; C₃-C₆-Alkenyl, insbesondere C₃-C₅-Alkenyl wie 2-Propenyl, 1-Methyl-2-propenyl, 1,1-Dimethyl-2-propenyl. Cycloaliphatische Reste sind beispielsweise Cyclopentyl und Cyclohexyl.

Durch die Verwendung von Magnesiumalkoholaten als Base wurden überraschend weitaus höhere Ausbeuten erzielt als nach literaturbekannten Verfahren, die Ausbeuten von nur 14 bis 44 % bei Verwendung von Natriumalkoholaten bzw. 21 bis 63 % bei Verwendung von Natriumhydrid mit sich bringen wie die Zusammenstellung in Tabelle I zeigt.

**Tabelle I**

| R¹ | R⁵ | R² | Base | Lösungsmittel | Ausbeute | Literatur |
|---|---|---|---|---|---|---|
| Me | Et | Et | NaOMe | MeOH | 44 % | 1) |
| Me | Me | Me | NaOMe | MeOH | 38 % | 2) |
| Me | Me | Et | NaH | Toluol | 63 % | 3) |
| Me | Me | Me | NaH | Benzol | 48 % | 3) |
| Me | Me | Me | NaH | Toluol | 46 % | 3) |
| Et | Me | Me | NaH | Toluol | 21 % | 3) |
| iPr | Me | Me | NaH | Toluol | 31 % | 3) |
| iPr | Me | Me | NaH | Benzol | 28 % | 3) |
| iPr | Me | Et | NaH | Toluol | 32 % | 3) |
| iPr | Me | Me | NaOMe | MeOH | 18 % | 2) |
| iPr | Me | Me | NaOMe | THF | 14 % | 2) |

| erfindungsgemäßes Verfahren | | | | | | |
|---|---|---|---|---|---|---|
| iPr | Me | Me | Mg(OMe)₂ | THF | 74 % | |
| iPr | Me | Et | Mg(OEt)₂ | THF | 78 % | |
| iPr | Me | Et | Mg(OEt)₂ | Toluol | 69 % | |
| THF = Tetrahydrofuran Me = Methyl; Et = Ethyl; i-Pr = Isopropyl 1) L. Panizzi, Gazz. Chim. Ital., 69, 322 (1939); 2) analog Lit. 1 durchgeführt 3) V. Maywald et al., DE-A-39 31 627 | | | | | | |

Als Magnesiumalkoholate kommen solche von C₁-C₆-Alkoholen wie Methanol, Ethanol, n-Propanol, Isopropanol, n-Butanol, iso-Butanol, n-Pentanol, iso-Pentanol, n-Hexanol oder Isohexanol sowie von C₃-C₆-Alkenolen wie Allylalkohol und Cycloalkylalkohole wie Cyclopentanol und Cyclohexanol in Betracht.

Besonders bevorzugt sind leicht zugängliche und billige Magnesiumalkoholate wie Magnesiummethylat, -ethylat und propylat, die kommerziell erhältlich sind.

Herstellverfahren für Erdalkalialkoholate sind literaturbekannt (siehe Houben-Weyl, Methoden der Organischen Chemie, 4. Auflage, Band VI/2, Sauerstoffverbindungen I, S. 15, Georg Thieme Verlag).

Die erfindungsgemäße Umsetzung erfolgt bevorzugt in der Weise, daß man die β-Ketosäure bzw. den β-Ketoester II in einem inerten organischen Lösungsmittel bei Temperaturen von -20°C bis zum Siedepunkt des organischen Lösungsmittels, vorzugsweise bei Temperaturen zwischen 0°C-50°C, mit der 1-5fach, bevorzugt 1-2 fach molaren Menge des Magnesiumalkoholats IV in das Magnesiumenolat überführt. Vorzugsweise wird dabei das Magnesiumalkoholat IV im Lösungsmittel vorgelegt und diese Mischung mit einer Lösung des Ausgangsstoffes II versetzt. Anschließend wird das Hydroxamsäurechlorid IIIa oder IIIb bei Temperaturen zwischen 0°C bis zum Siedepunkt des organischen Lösungsmittels, vorzugsweise bei 20-70°C zugetropft. Um die Bildung von Furoxanen, die durch Dimerisierung der Hydroxamsäurechloride III oder deren Folgeprodukte entstehen können, zu vermeiden, sollte die Zugabe langsam, d.h. innerhalb von 1-12 Stunden, vorzugsweise 3-8 Stunden, erfolgen, wodurch die Konzentration niedrig gehalten wird.

Als organisches Lösungsmittel eignen sich ganz allgemein Kohlenwasserstoffe wie Pentan, Hexan, Heptan, Petrolether, Ligroin oder Cyclohexan, Halogenkohlenwasserstoffe wie Dichlormethan, Tetrachlorkohlenstoff und Chlorbenzol, Nitrile wie Acetonitril, Butyronitril und Benzonitril, Amide wie Formamid, Methylformamid, Dimethylformamid und Diethylformamid, Sulfoxide und Sulfone wie Dimethylsulfoxid und Sulfolan. Insbesondere eignen sich Ether wie Diethylether, Ethyl-n-propylether, Di-n-butylether, Diisoamylether, Cyclohexylmethylether, Methyl-tert.-butylether, Diisopropylether, Ethylenglykoldimethylether, Diethylenglykoldimethylether, Tetrahydrofuran, Dioxan, Anisol, Phenetol oder aromatische Kohlenwasserstoffe wie Benzol, Toluol oder Xylol. Selbstverständlich können auch Gemische dieser Lösungsmittel verwendet werden.

Die Menge an Lösungsmittel ist nicht kritisch. Normalerweise verwendet man die 1-20-fache, vorzugsweise die 1-10-fache Gewichtsmenge an Lösungsmittel, bezogen auf die Gewichtsmenge β-Ketosäure bzw. β-Ketoester II.

Nach erfolgter Umsetzung (nach ca. 1-24 Stunden) säuert man die Lösung mit einer geeigneten Säure an und erhitzt das Reaktionsgemisch 1-6 Stunden unter Rückfluß. Dabei kann von Vorteil sein, das bei der stattfindenden Cyclisierung entstehende Wasser aus dem Reaktionsgemisch zu entfernen. Bei mit Wasser nicht mischbaren Lösungsmitteln verwendet man dazu zweckmäßigerweise einen Wasserabscheider, bei mit Wasser mischbaren Lösungsmitteln einen Soxhlet-Extraktor, dessen Extraktionshülse mit einem geeigneten Trockenmittel, beispielsweise Molekularsieb, gefüllt ist. Das Reaktionsprodukt I kann dann wie üblich durch Zugabe von Wasser und anschließende Extraktion mit einem organischen Lösungsmittel isoliert werden.

Als Säuren verwendet man zweckmäßigerweise Carbon- und Sulfonsäuren wie Ameisensäure, Essigsäure, Propionsäure, Oxalsäure, Methansulfonsäure oder p-Toluolsulfonsäure, anorganische Säuren wie Schwefelsäure, Salzsäure, Phosphorsäure sowie Salze wie Ammoniumchlorid und Aminhydrochloride.

Besondere Bedingungen bezüglich des Druckes sind nicht erforderlich, im allgemeinen nimmt man die Umsetzung bei Atmosphärendruck vor.

Das Verfahren kann sowohl kontinuierlich als auch diskontinuierlich durchgeführt werden. Als ein Beispiel der kontinuierlichen Arbeitsweise sei die Umsetzung der Reaktionspartner in einem Rohrreaktor genannt.

Im Hinblick auf die bestimmungsgemäße Verwendung der Zwischenprodukte I haben die Substituenten R¹, R² und A insbesondere die folgende Bedeutung:
- R¹: Wasserstoff;
ein niedermolekularer Alkylrest wie C₁-C₆-Alkyl, insbesondere C₁-C₄-Alkyl wie Methyl, Ethyl, Propyl, Isopropyl, Butyl, Isobutyl, sek.-Butyl, tert.-Butyl, das durch ein bis drei Halogenatome wie Fluor, Chlor, Brom, Iod, insbesondere Fluor und Chlor, einen C₁-C₃-Alkoxyrest wie Methoxy, Ethoxy, Propoxy und Isopropoxy, insbesondere Methoxy und Ethoxy oder einen C₃-C₆-Cycloalkylrest wie Cyclopropyl, Cyclobutyl, Cyclopentyl und Cyclohexyl, insbesondere Cyclopropyl, substituiert sein kann;
ein Cycloalkylrest wie C₃-C₈-Cycloalkyl, z.B. Cyclopropyl, Cyclobutyl, Cyclopentyl, Cyclohexyl, Cycloheptyl, Cyclooctyl, insbesondere Cyclopropyl, das ein- bis dreimal durch C₁-C₄-Alkyl wie vorstehend genannt, insbesondere Methyl, substituiert sein kann;
ein 5- bis 6-gliedriger gesättigter, ungesättigter oder aromatischer heterocyclischer Rest, enthaltend ein oder zwei Heteroatome, ausgewählt aus der Gruppe Sauerstoff, Schwefel und Stickstoff wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 2-Oxazolyl, 4-Oxazolyl, 5-Oxazolyl, 2-Thiazolyl, 4-Thiazolyl, 5-Thiazolyl, 2-Pyridyl, 3-Pyridyl, 4-Pyridyl, der durch C₁-C₃-Alkyl wie vorstehend genannt, insbesondere Methyl, C₁-C₃-Alkoxy wie Methoxy, Ethoxy, Propoxy und Isopropoxy, insbesondere Methoxy oder Halogen wie Fluor, Chlor, Brom und Iod, insbesondere Fluor und Chlor, substituiert sein kann;
Phenyl, das eine bis drei der folgenden Gruppen tragen kann: C₁-C₄-Alkyl wie vorstehend genannt, insbesondere Methyl, C₁-C₄-Halogenalkyl, insbesondere Trifluormethyl, C₁-C₄-Alkoxy wie vorstehend genannt, insbesondere Methoxy, C₁-C₄-Halogenalkoxy, insbesondere Trifluormethoxy, Halogen wie vorstehend genannt, insbesondere Fluor und Chlor, Nitro und Cyano;
- R²: Wasserstoff; Alkyl wie unter R¹ genannt, z.B. C₁-C₄-Alkyl, insbesondere Methyl und Ethyl, Cycloalkyl wie unter R¹ genannt, insbesondere Cyclohexyl, Benzyl und C₃-C₆-Alkenyl, insbesondere Allyl;
- A: eine Aminogruppe NR³R⁴, worin
- R³: Wasserstoff,
einen aliphatischen Alkyl-, Alkenyl- oder Alkinylrest wie C₁-C₆-Alkyl, bevorzugt C₁-C₃-Alkyl wie vorstehend genannt, insbesondere Methyl, Ethyl und Isopropyl, C₃-C₆-Alkenyl, insbesondere C₃-C₅-Alkenyl wie 2-Propenyl, 1-Methyl-2-propenyl, 1,1-Dimethyl-2-propenyl, C₃-C₆-Alkinyl, insbesondere C₃-C₅-Alkinyl wie 2-Propinyl, 1-Methyl-2-propinyl, 1,1-Dimethyl-2-propinyl, C₃-C₆-Cycloalkyl wie vorstehend genannt, insbesondere Cyclopropyl und
- R⁴: einen aliphatischen, z.B. ggf. substituierten Alkyl-, Alkenyl- oder Alkinylrest wie C₁-C₆-Alkyl, insbesondere C₁-C₄-Alkyl wie Methyl, Ethyl, Propyl, Isopropyl, Butyl, Isobutyl, sek-Butyl, tert.-Butyl, das einmal durch C₁-C₄-Alkoxy wie Methoxy, Ethoxy, Propoxy, Isopropoxy, Butoxy, Isobutoxy, sek-Butoxy, tert.-Butoxy, insbesondere Methoxy und Ethoxy, C₁-C₄-Alkylthio wie Methylthio, Ethylthio, Propylthio, Isopropylthio, Butylthio, Isobutylthio, sek-Butylthio, tert.-Butylthio, insbesondere Methylthio und Ethylthio, Halogen wie unter R¹ genannt, insbesondere Fluor und Chlor, C₃-C₆-Cycloalkyl wie unter R¹ genannt, insbesondere Cyclopropyl, Cyano oder Phenyl substituiert sein kann;
C₃-C₆-Alkenyl, insbesondere C₃-C₅-Alkenyl wie 2-Propenyl, 1-Methyl-2-propenyl, 1,1-Dimethyl-2-propenyl,
C₃-C₆-Alkinyl, insbesondere C₃-C₅-Alkinyl wie 2-Propinyl, 1-Methyl-2-propinyl, 1,1-Dimethyl-2-propinyl,
ein cycloaliphatischer Rest, z.B. C₃-C₆-Cycloalkyl wie unter R¹ genannt, insbesondere Cyclopropyl, Cyclopentyl und Cyclohexyl, das bis zu zweimal durch C₁-C₄-Alkyl wie vorstehend genannt, insbesondere Methyl und Ethyl oder Halogen wie unter R¹ genannt, insbesondere Fluor und Chlor, substituiert sein kann;
Phenyl, das einen bis drei der folgenden Reste tragen kann: C₁-C₄-Alkyl wie unter R¹ genannt, insbesondere Methyl, C₁-C₄-Halogenalkyl, insbesondere Trifluormethyl, C₁-C₄-Alkoxy, insbesondere Methoxy, Halogen wie unter R¹ genannt, insbesondere Fluor und Chlor, Cyano, Nitro,
oder
- R³,R⁴: gemeinsam eine Methylenkette mit 4 bis 7 Gliedern, welche durch Sauerstoff, Schwefel oder N-Methyl unterbrochen sein kann,
oder A steht für eine Gruppe OR⁵, wobei
- R⁵: C₁-C₄-Alkyl wie unter R¹ genannt, insbesondere Methyl und Ethyl, C₃-C₆-Cycloalkyl wie unter R¹ genannt, insbesondere Cyclohexyl, Benzyl und C₃-C₆-Alkenyl, insbesondere Allyl bedeutet.

Die für das erfindungsgemäße Verfahren benötigten β-Ketosäuren bzw. β-Ketoester II sind entweder kommerziell erhältlich oder nach allgemein bekannten Verfahren herstellbar (z.B. Org. Synth., 61, p. 5 (1982); Org. Synth. Coll. Vol. 4, p. 415 (1963); J. Org. Chem., 44, p. 310 (1979); J. Org. Chem., 43, p. 2087 (1978); Synthesis, 1978, p. 829; Org. Prep. Proced Int., 10, p. 221 (1978)).

Die Hydroxamsäurechloride IIIb sind literaturbekannt (vgl. z.B. DE-A1-28 17 838; J. Org. Chem., Vol. 48, No. 3, 1983; US 3,557,190).

Die Hydroxamsäurechloride IIIa sind beispielsweise nach dem in der Auslegeschrift DE 19 63 061 beschriebenen Verfahren ausgehend von Acetoacetamiden herstellbar. Nach diesem allgemeinen Verfahren lassen sich prinzipiell auch Hydroxamsäurechloride, die bisher nicht in der Literatur beschrieben sind, herstellen. Ein Beispiel ist nachstehend beschrieben.

### Beispiele

### Herstellung von Hydroxamsäurechloriden IIIa

Cyclopropylcarbamoylformhydroxamoylchlorid 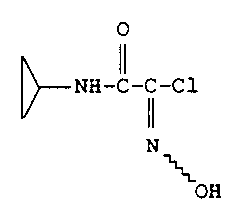 28,5 g (0,5 mol) Cyclopropylamin werden in 500 ml Wasser bei Raumtemperatur vorgelegt und anschließend 42,0 g (0,5 mol) Diketen zugetropft. Dabei sinkt der pH-Wert von 12.0 auf 5.5-6.5 ab. Man rührt 10 min nach, versetzt mit 37,9 g (0,55 mol) Natriumnitrit und gibt anschließend ca. 75 ml konz. Salzsäure so zu, daß der pH-Wert stets oberhalb von 4.5 bleibt. Nach vollendeter Zugabe werden bei Raumtemperatur 41,1 g (0,58 mol) elementares Chlor eingegast. Der vollständige Umsatz wird mittels DC oder RPLC kontrolliert. Das bei der Reaktion entstehende Hydroxamsäurechlorid kann entweder durch Filtration bei 0°C oder durch mehrfache Extraktion mit je 200 ml Ethylacetat und Abziehen des Solvens im Vakuum isoliert werden. Der so erhaltene Feststoff wird mit Wasser gewaschen und bei maximal 40°C im Vakuum getrocknet. Man erhält 72,1 g (89 %) Cyclopropylcarbamoylformhydroxamoylchlorid als weißen Feststoff.
¹H-NMR (250 MHz, DMSO): δ = 0,50-0,78 (m; 4H), 2,75 (m; 1H), 8,48 (d; 1H, NH), 12,80 (s; 1H, OH)

Allgemeine Vorschrift zur Herstellung von Isoxazol-3-carbonsäureamid-4-carbonsäureestern Ia via Umsetzung von β-Ketoestern IIa mit Hydroxamsäurechloriden IIIa in Anwesenheit von Magnesiumalkoholaten. 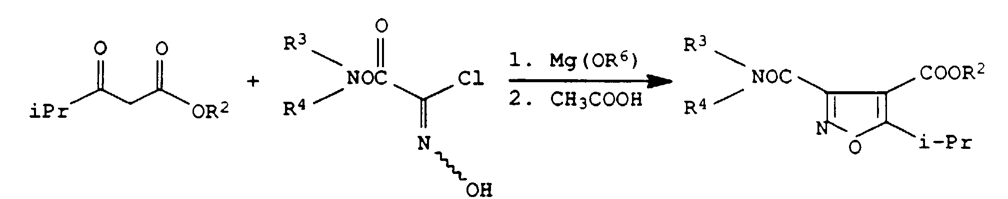 R² = R⁶ = Methyl oder Ethyl
0,31 mol Magnesiumspäne läßt man unter Feuchtigkeitsausschuß mit 50 ml trockenem Alkohol (R⁶OH) reagieren. Die Reaktion wird durch Zugabe weniger Tropfen Brom oder Tetrakohlenstoff bzw. einer Spatelspitze Jod in Gang gebracht. Nach Beendigung der Reaktion wird der überschüssige Alkohol im Vakuum entfernt. Zu einer Suspension des auf diese Weise hergestellten Magnesiumalkoholats in 400 ml eines trockenen, inerten organischen Lösungsmittels werden anschließend 0,3 mol β-Ketoester II in 50 ml Lösungsmittel bei 10 bis 20°C zugetropft. Man rührt ca. 3 h bei Raumtemperatur. Zu der Lösung des gebildeten Magnesiumenolats werden langsam 0.3 mol Hydroxamsäurechlorid IIIa in 300 ml trockenem inerten organischen Lösungsmittel bei Raumtemperatur zugegeben. Die Zugabe erfolgt über 5 Stunden. Man läßt 4 bis 12 h bei Raumtemperatur nachrühren, säuert die Lösung durch Zugabe von ca. 45 g Essigsäure an und erhitzt 1 - 4 h unter Rückfluß. Der vollständige Umsatz zum Cyclisierungsprodukt wird mittels HPLC-Analytik kontrolliert. Man läßt abkühlen, nimmt das Reaktionsgemisch in einem Gemisch aus Wasser und Ethylacetat auf, trennt die Phasen, extrahiert die wässrige Phase mit Ethylacetat, trocknet die vereinigten organischen Phasen und zieht die Solventien am Rotationsverdampfer ab. Falls nötig wird das so erhaltene Rohprodukt durch Säulenfiltration an Kieselgel (Cyclohexan/Ethylacetat) gereinigt.

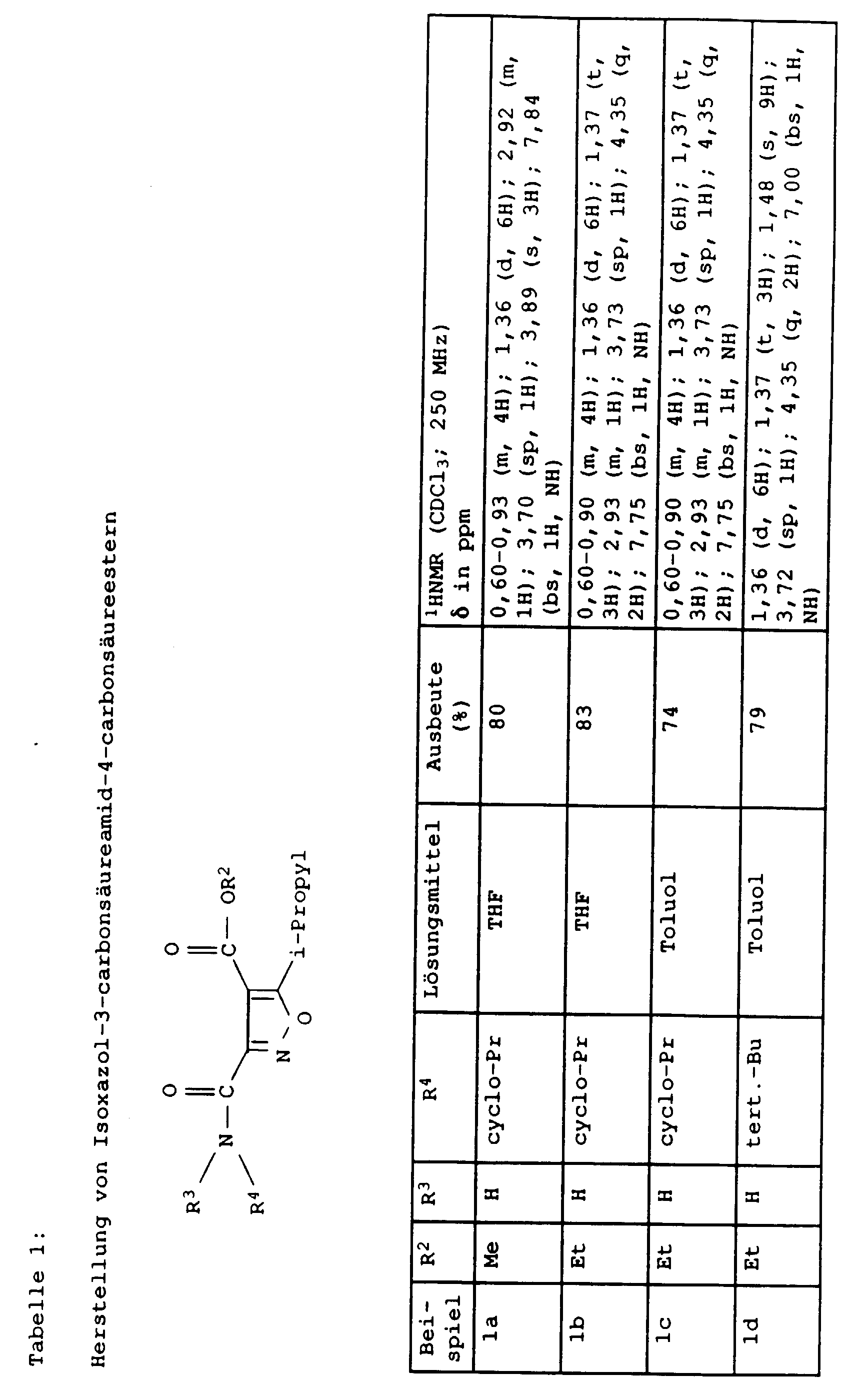

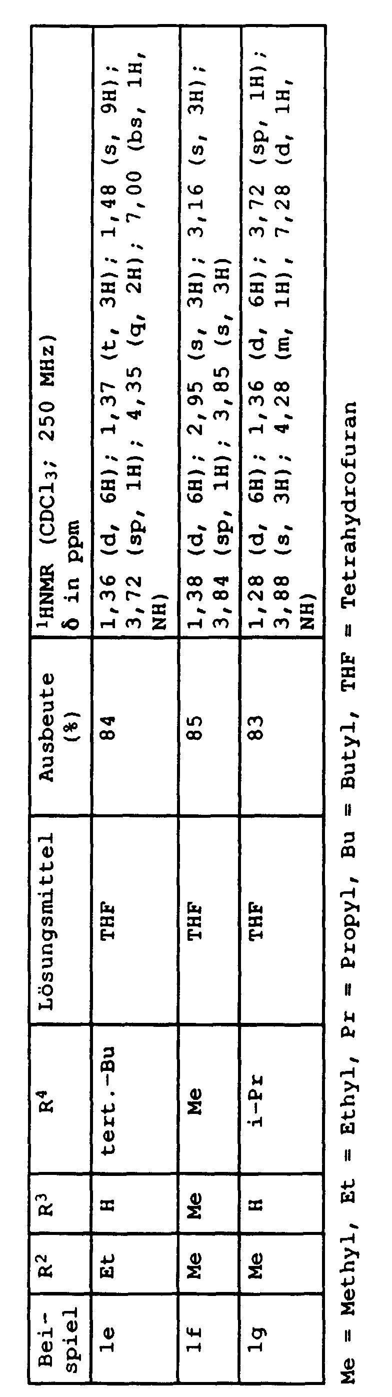

Allgemeine Vorschrift zur Herstellung von Isoxazol-3,4-dicarbonsäureestern Ib via Umsetzung von β-Ketoestern IIa mit Hydroxamsäurechloriden IIIb in Anwesenheit von Magnesiumalkoholaten. 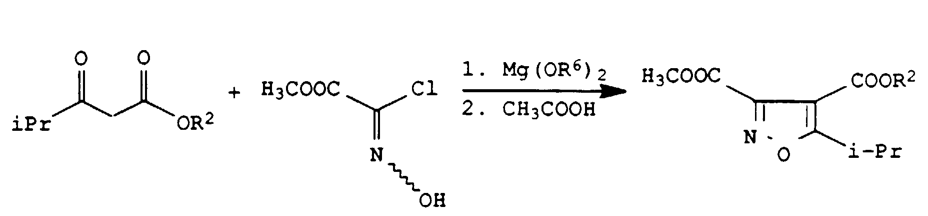 R² = R⁶ = Methyl oder Ethyl
Zu einer Lösung von 0,31 mol kommerziell erhältlichem Magnesiummethylat oder -ethylat in 400 ml trockenem inerten organischen Lösungsmittel werden 0,3 mol β-Ketoester IIa bei 10 bis 20°C zugegeben. Man rührt ca. 3 h bei Raumtemperatur. Zu der Lösung des gebildeten Magnesiumenolats werden langsam 0,3 mol Hydroxamsäurechlorid IIIb in 300 ml trockenem, inerten organischen Lösungsmittel bei Raumtemperatur zugegeben. Die Zugabe erfolgt über 5 Stunden. Man läßt 4 bis 12 h bei Raumtemperatur nachrühren, säuert die Lösung durch Zugabe von 45 g Essigsäure an, erhitzt 1 - 4 h unter Rückfluß und verfährt weiter wie vorstehend beschrieben.

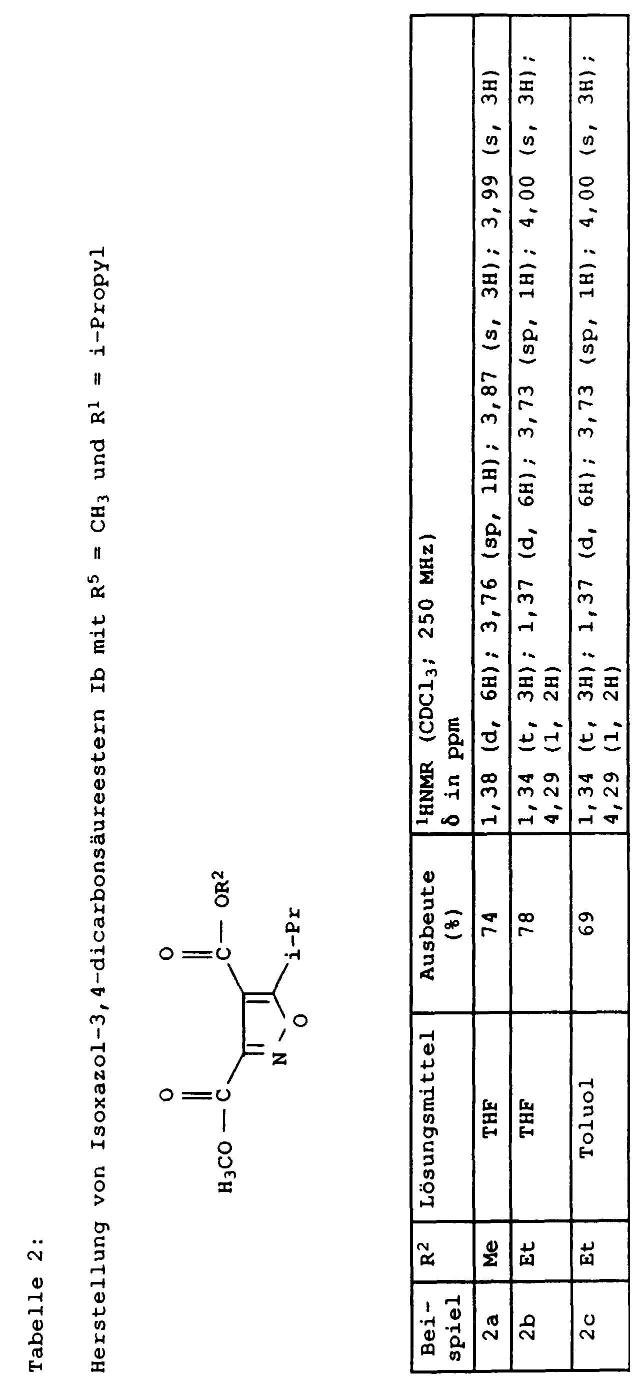

### Vergleichsbeispiele:

### Beispiel I:

### 5-Methylisoxazol-3,4-dicarbonsäuredimethylester

Zu einer Lösung von 10,8 g (0,2 mol) Natriummethylat (krist.) in 150 ml trockenem Methanol gibt man bei 0 - 10°C 23,2 g (0,2 mol) Acetessigsäuremethylester in 50 ml absoluten Methanol und rührt 3 h bei dieser Temperatur. Anschließend tropft man bei 0 - 10°C langsam 27,5 g (0,2 mol) Methyl-a-chloro-a-oximinoacetat in 100 ml absoluten Methanol zu und rührt 12 h bei Raumtemperatur. Zur Aufarbeitung engt man die Lösung weitgehend ein, gibt 800 ml Wasser zu, wobei sich das Produkt als Öl absetzt. Die organische Phase wird abgetrennt, die wässrige Phase mit Dichlormethan extrahiert, die vereinigten organischen Phasen getrocknet und das Solvens am Rotationsverdampfer abgezogen. Der Rückstand wird durch Säulenchromatographie an Kieselgel (Lösungsmittel Cyclohexan/Ethylacetat 3:1) gereinigt. Man erhält 15,1 g (38 % d. Theorie) 5-Methylisoxazol-3,4-dicarbonsäuredimethylester.
¹HNMR (250 MHz; CDCl₃) d = 2.72 (s;3H), 3.87 (s;3H), 4.00 (s;3H)

### Beispiel II:

Zu 9,9 g (0,33 mol) Natriumhydrid (100 %) in 500 ml trockenem Toluol bzw. Benzol tropft man bei Raumtemperatur 0,3 mol β-Ketoester in 100 ml Lösungsmittel und rührt 3 h. Anschließend gibt man 0,3 mol Methyl-a-chloro-a-oximinoacetat in 200 ml Lösungsmittel zu und rührt 12 h bei Raumtemperatur. Danach überführt man das Reaktionsgemisch in eine Soxhletapparatur (Extraktionshülse gefüllt mit Molekularsieb 4 Å) fügt 3 g Methansulfonsäure hinzu und erhitzt 1 - 4 h unter Rückfluß. Man läßt abkühlen, wäscht die organische Phase einmal mit Wasser und zieht das Solvens im Vakuum ab. Gegebenenfalls wird das Rohprodukt durch Säulenfiltration an Kieselgel (Lösungsmittel Cyclohexan/Ethylacetat) gereinigt. Die Ergebnisse der verschiedenen Versuche sind in Tabelle 3 aufgeführt.

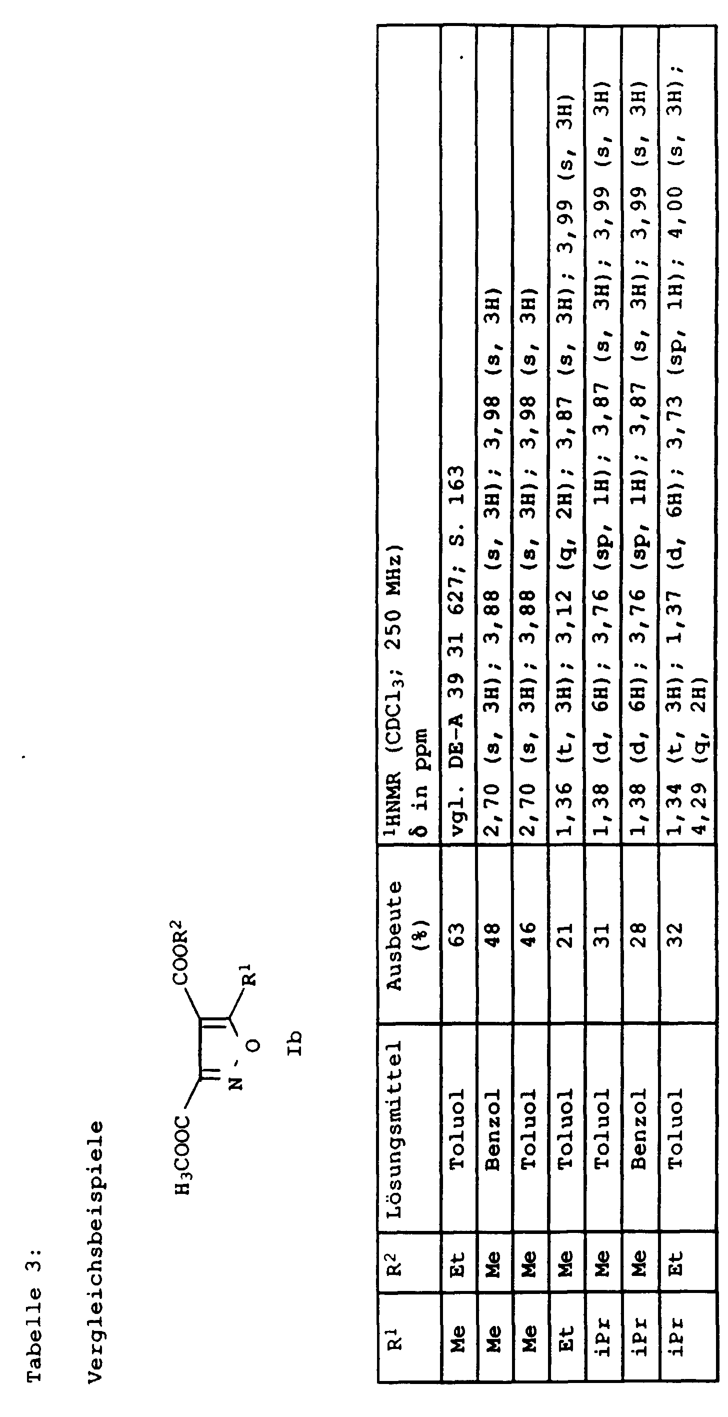

## Patentansprüche

1. Verfahren zur Herstellung von Isoxazol-3,4-dicarbonsäurederivaten der Formel I 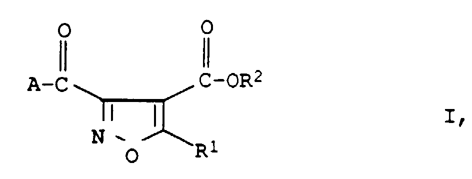 in der die Substituenten die folgende Bedeutung haben:
R¹ Wasserstoff, ein Alkyl-, Cycloalkyl- oder Phenylrest oder ein 5- bis 6-gliedriger heterocyclischer Rest, wobei die organischen Reste unter den Reaktionsbedingungen inerte Substituenten tragen können;
R² Wasserstoff, ein Alkyl-, Cycloalkyl-, Benzyl- oder C₃-C₆-Alkenylrest;
A eine Aminogruppe NR³R⁴, in der R³ für Wasserstoff, einen aliphatischen oder cycloaliphatischen Rest steht und R⁴ einen aliphatischen, einen cycloaliphatischen oder einen ggf. substituierten Phenylrest bedeutet oder R³ zusammen mit R⁴ eine 4- bis 7-gliedrige Alkylenkette bildet, die durch Sauerstoff, Schwefel oder N-Methyl unterbrochen sein kann oder
eine Gruppe OR⁵, in der R⁵ für einen Alkyl-, Cycloalkyl-, Benzyl- oder Alkenylrest steht;
durch Deprotonierung einer CH-aciden Verbindung der Formel II 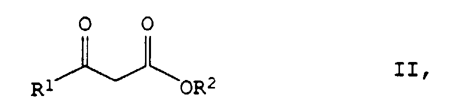 mittels einer Base und Umsetzung mit Hydroxamsäurechloriden der Formel III 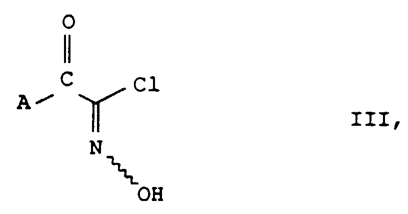 dadurch gekennzeichnet, daß man zur Deprotonierung ein Magnesiumalkoholat der Formel IV
Mg(OR⁶)₂ IV,
in der R⁶ einen aliphatischen oder cycloaliphatischen Rest bedeutet, verwendet.

2. Verfahren nach Anspruch 1, dadurch gekennzeichnet, daß man Magnesiummethylat oder Magnesiumethylat als Base verwendet.

3. Verfahren nach den Ansprüchen 1 und 2, dadurch gekennzeichnet, daß man die Base in einer Menge von 1- bis 5 mol, bezogen auf den β-Ketoester II (R² ≠ H) bzw. 2- bis 5 mol, bezogen auf die β-Ketosäure II (R² = H) verwendet.

4. Verfahren nach den Ansprüchen 1 bis 3, dadurch gekennzeichnet, daß man die Umsetzung in Gegenwart eines aromatischen Kohlenwasserstoffs oder eines Ethers als Lösungsmittel vornimmt.

5. Verfahren nach Anspruch 4, dadurch gekennzeichnet, daß man Toluol oder Tetrahydrofuran als Lösungsmittel verwendet.

## Claims

1. A process for preparing isoxazole-3,4-dicarboxylic acid derivatives of the formula I 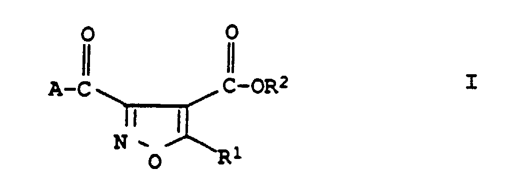 where
R¹ is hydrogen, alkyl, cycloalkyl or phenyl, or a 5- to 6-membered heterocyclic radical, and the organic radicals can have substituents which are inert under the reaction conditions;
R² is hydrogen, alkyl, cycloalkyl, benzyl or C₃-C₆-alkenyl;
A is NR³R⁴ where R³ is hydrogen or an aliphatic or cycloaliphatic radical and R⁴ is an aliphatic or cycloaliphatic radical or unsubstituted or substituted phenyl, or R³ forms together with R⁴ a 4- to 7-membered alkylene chain which can be interrupted by oxygen, sulfur or N-methyl, or
is OR⁵ where R⁵ is alkyl, cycloalkyl, benzyl or alkenyl;
by deprotonation of a CH-acid compound of the formula II 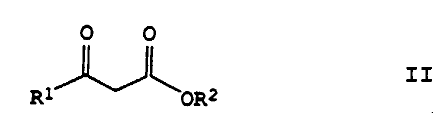 using a base and reacting with hydroxamyl chlorides of the formula III 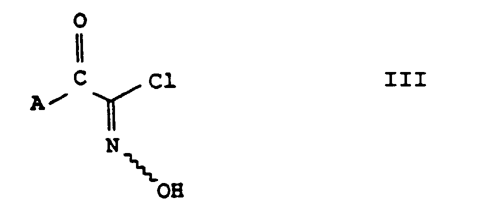 wherein a magnesium alcoholate of the formula IV
Mg(OR⁶)₂ IV
where R⁶ is an aliphatic or cycloaliphatic radical is used for the deprotonation.

2. A process as claimed in claim 1, wherein magnesium methoxide or magnesium ethoxide is used as base.

3. A process as claimed in claims 1 and 2, wherein the base is used in an amount of from 1 to 5 mol per mol of β-keto ester II (R² ≠ H) or from 2 to 5 mol per mol of β-keto acid II (R² = H).

4. A process as claimed in claims 1 to 3, wherein the reaction is carried out in the presence of an aromatic hydrocarbon or of an ether as solvent.

5. A process as claimed in claim 4, wherein toluene or tetrahydrofuran is used as solvent.

## Revendications

1. Procédé de préparation de dérivés d'acides isoxazole-3,4-dicarboxyliques de formule I 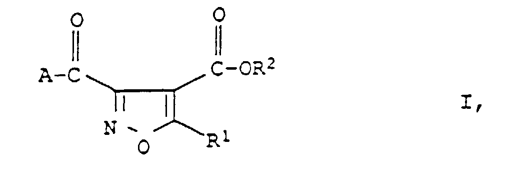 dans laquelle les symboles ont les significations suivantes :
R¹ : l'hydrogène, un groupe alkyle, cycloalkyle ou phényle ou un groupe hétérocyclique à cinq ou six chaînons, les groupes organiques pouvant porter des substituants inertes dans les conditions de la réaction ;
R² : l'hydrogène, un groupe alkyle, cycloalkyle, benzyle ou alcényle en C3-C6 ;
A : un groupe amino NR³R⁴ dans lequel R³ représente l'hydrogène, un groupe aliphatique ou cycloaliphatique et R⁴ un groupe aliphatique, un groupe cycloaliphatique ou un groupe phényle éventuellement substitué, ou bien R³ forme avec R⁴ une chaîne alkylène de quatre à sept chaînons qui peut être interrompue par l'oxygène, le soufre où le groupe N-méthyle, ou bien
un groupe OR⁵ dans lequel R⁵ représente un groupe alkyle, cycloalkyle, benzyle ou alcényle ;
par déprotonation d'un composé CH acide de formule II 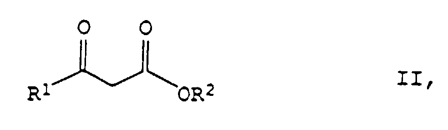 à l'aide d'une base, et réaction avec des chlorures d'acides hydroxamiques de formule III 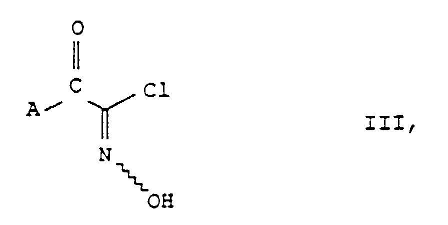 ce procédé se caractérisant par le fait que l'on utilise pour la déprotonation un alcoolate de magnésium de formule IV
Mg(OR⁶)₂ IV,
dans laquelle R⁶ représente un radical aliphatique ou cycloaliphatique.

2. Procédé selon la revendication 1, caractérisé par le fait que la base utilisée est le méthylate de magnésium ou l'éthylate de magnésium.

3. Procédé selon les revendications 1 et 2, caractérisé par le fait que l'on utilise la base en quantité de 1 à 5 mol par rapport au β-cétoester II (R² ≠ H) ou de 2 à 5 mol par rapport au β-cétoacide II (R² = H).

4. Procédé selon les revendications 1 à 3, caractérisé par le fait que l'on effectue la réaction en présence d'un solvant consistant en un hydrocarbure aromatique ou un éther.

5. Procédé selon la revendication 4, caractérisé par le fait que l'on utilise en tant que solvant le toluène ou le tétrahydrofuranne.
